# EUROPEAN PATENT APPLICATION

(11) **EP 0 565 370 A1**
(43) Date of publication of application: **13.10.1993**
(21) Application number: 93302759.1
(22) Date of filing: 07.04.1993
(51) Int. Cl.: C07K 13/00, C12N 15/12, C12N 15/70, C12N 5/10

(54) **Serotonin receptor protein and related DNA compounds**

(30) Priority: 09.04.1992 US 864005
(71) Applicant: ELI LILLY AND COMPANY, Indianapolis, Indiana 46285 (US)
(72) Inventor: Baez, Melvyn, Indianapolis, Indiana 46208 (US); Kursar, Jonathan David, Indianapolis, Indiana 46268 (US)
(74) Representative: Hudson, Christopher Mark

(57) **Abstract**

This invention provides a serotonin receptor protein and related DNA compounds. The invention will further the characterization of serotonin-related behavior and disease.

### SEQUENCE LISTING

(1) GENERAL INFORMATION:
   (i) APPLICANT:
      (A) NAME: ELI LILLY AND COMPANY
      (B) STREET: ERL WOOD MANOR
      (C) CITY: WINDLESHAM
      (D) STATE: SURREY
      (E) ZIP: GU20 6PH
   (ii) TITLE OF INVENTION: SEROTONIN RECEPTOR PROTEIN AND RELATED DNA COMPOUNDS
   (iii) NUMBER OF SEQUENCES: 5
   (iv) COMPUTER READABLE FORM:
      (A) MEDIUM TYPE: Floppy disk
      (B) COMPUTER: Macintosh
      (C) OPERATING SYSTEM: Macintosh 7.0
      (D) SOFTWARE: Microsoft Word
(2) INFORMATION FOR SEQ ID NO:1:
   (i) SEQUENCE CHARACTERISTICS:
      (A) LENGTH: 1437 base pairs
      (B) TYPE: nucleic acid
      (C) STRANDEDNESS: double
      (D) TOPOLOGY: linear
   (ii) MOLECULE TYPE: cDNA
   (iii) HYPOTHETICAL: NO
   (iv) ANTI-SENSE: NO
   (ix) FEATURE:
      (A) NAME/KEY: CDS
      (B) LOCATION: 1..1437
   (xi) SEQUENCE DESCRIPTION: SEQ ID NO:1:
   ATG GCT TCA TCT TAT AAA ATG TCT GAA CAA AGC ACA ATT TCT GAG CAC        48
   Met Ala Ser Ser Tyr Lys Met Ser Glu Gln Ser Thr Ile Ser Glu His
     1               5                  10                  15
   ATT TTG CAG AAA ACA TGT GAT CAC CTG ATC TTG ACT GAC CGT TCT GGA        96
   Ile Leu Gln Lys Thr Cys Asp His Leu Ile Leu Thr Asp Arg Ser Gly
                20                  25                  30
   TTA AAG GCA GAA TCA GCA GCA GAG GAA ATG AAG CAG ACT GCC GAG AAC       144
   Leu Lys Ala Glu Ser Ala Ala Glu Glu Met Lys Gln Thr Ala Glu Asn
            35                  40                  45
   CAG GGG AAT ACA GTG CAC TGG GCA GCT CTC CTG ATC TTC GCG GTA ATA       192
   Gln Gly Asn Thr Val His Trp Ala Ala Leu Leu Ile Phe Ala Val Ile
        50                  55                  60
   ATC CCC ACC ATT GGC GGG AAC ATC CTG GTT ATT CTG GCT GTT TCA CTG       240
   Ile Pro Thr Ile Gly Gly Asn Ile Leu Val Ile Leu Ala Val Ser Leu
    65                  70                  75                  80
   GAG AAA AGG CTG CAG TAC GCT ACC AAC TAC TTT CTA ATG TCC TTG GCG       288
   Glu Lys Arg Leu Gln Tyr Ala Thr Asn Tyr Phe Leu Met Ser Leu Ala
                        85                      90                      95
   GTG GCT GAT TTG CTG GTT GGA TTG TTT GTG ATG CCG ATT GCT CTC TTA      336
   Val Ala Asp Leu Leu Val Gly Leu Phe Val Met Pro Ile Ala Leu Leu
               100                 105                 110
   ACA ATC ATG TTT GAG GCT ACA TGG CCC CTC CCA CTG GCC CTG TGT CCT      384
   Thr Ile Met Phe Glu Ala Thr Trp Pro Leu Pro Leu Ala Leu Cys Pro
           115                 120                 125
   GCC TGG TTA TTC CTT GAT GTT CTC TTT TCA ACT GCC TCC ATC ATG CAT      432
   Ala Trp Leu Phe Leu Asp Val Leu Phe Ser Thr Ala Ser Ile Met His
       130                 135                 140
   CTC TGT GCC ATT TCC CTG GAT CGC TAT ATA GCC ATC AAA AAG CCA ATT      480
   Leu Cys Ala Ile Ser Leu Asp Arg Tyr Ile Ala Ile Lys Lys Pro Ile
   145                 150                 155                 160
   CAG GCC AAT CAG TGC AAT TCC CGG ACT ACT GCA TTC GTC AAG ATT ACG      528
   Gln Ala Asn Gln Cys Asn Ser Arg Thr Thr Ala Phe Val Lys Ile Thr
                   165                 170                 175
   GTG GTA TGG TTA ATT TCA ATA GGC ATC GCC ATC CCA GTC CCT ATT AAA      576
   Val Val Trp Leu Ile Ser Ile Gly Ile Ala Ile Pro Val Pro Ile Lys
               180                 185                 190
   GGA ATA GAG GCT GAT GTG GTC AAC GCA CAC AAC ATC ACC TGT GAG CTG      624
   Gly Ile Glu Ala Asp Val Val Asn Ala His Asn Ile Thr Cys Glu Leu
           195                 200                 205
   ACA AAG GAC CGC TTT GGC AGT TTC ATG CTC TTT GGG TCA CTG GCT GCT      672
   Thr Lys Asp Arg Phe Gly Ser Phe Met Leu Phe Gly Ser Leu Ala Ala
       210                 215                 220
   TTC TTT GCA CCT CTC ACC ATC ATG ATA GTC ACC TAC TTT CTC ACC ATT      720
   Phe Phe Ala Pro Leu Thr Ile Met Ile Val Thr Tyr Phe Leu Thr Ile
   225                 230                 235                 240
   CAC GCT TTG CGG AAG AAA GCT TAC TTG GTC AGA AAC AGG CCA CCT CAA      768
   His Ala Leu Arg Lys Lys Ala Tyr Leu Val Arg Asn Arg Pro Pro Gln
                   245                 250                 255
   CGC CTA ACA CGG TGG ACT GTG TCC ACA GTT CTC CAA AGG GAA GAC TCA      816
   Arg Leu Thr Arg Trp Thr Val Ser Thr Val Leu Gln Arg Glu Asp Ser
               260                 265                 270
   TCC TTT TCA TCA CCA GAA AAG ATG GCG ATG CTG GAT GGC TCT CAC AAG      864
   Ser Phe Ser Ser Pro Glu Lys Met Ala Met Leu Asp Gly Ser His Lys
           275                 280                 285
   GAT AAA ATT CTA CCT AAC TCA ATT GAT GAG ACA CTG ATG AGA AGA ATG      912
   Asp Lys Ile Leu Pro Asn Ser Ile Asp Glu Thr Leu Met Arg Arg Met
       290                 295                 300
   TCC TCA GCA GGA AAA AAA CCA GCC CAG ACC ATT TCT AAT GAA CAG AGA      960
   Ser Ser Ala Gly Lys Lys Pro Ala Gln Thr Ile Ser Asn Glu Gln Arg
   305                 310                 315                 320
   GCC TCA AAG GTC CTT GGA ATT GTG TTT CTC TTC TTT CTG CTT ATG TGG     1008
   Ala Ser Lys Val Leu Gly Ile Val Phe Leu Phe Phe Leu Leu Met Trp
                   325                 330                 335
   TGC CCC TTT TTC ATT ACA AAC GTA ACT TTA GCT CTG TGT GAT TCC TGC     1056
   Cys Pro Phe Phe Ile Thr Asn Val Thr Leu Ala Leu Cys Asp Ser Cys
               340                 345                 350
   AAC CAG ACT ACT CTC AAA ACA CTC CTG CAG ATA TTT GTG TGG GTA GGC      1104
   Asn Gln Thr Thr Leu Lys Thr Leu Leu Gln Ile Phe Val Trp Val Gly
           355                 360                 365
   TAC GTT TCC TCG GGA GTG AAT CCT TTG ATC TAT ACC CTC TTC AAT AAG      1152
   Tyr Val Ser Ser Gly Val Asn Pro Leu Ile Tyr Thr Leu Phe Asn Lys
       370                 375                 380
   ACA TTT CGG GAA GCA TTT GGC AGG TAC ATC ACC TGC AAT TAC CAG GCC      1200
   Thr Phe Arg Glu Ala Phe Gly Arg Tyr Ile Thr Cys Asn Tyr Gln Ala
   385                 390                 395                 400
   ACA AAG TCA GTA AAA GTG CTT AGA AAG TGT TCT AGT ACA CTC TAT TTT      1248
   Thr Lys Ser Val Lys Val Leu Arg Lys Cys Ser Ser Thr Leu Tyr Phe
                   405                 410                 415
   GGG AAT TCA ATG GTA GAA AAC TCT AAA TTT TTC ACA AAA CAT GGA ATT      1296
   Gly Asn Ser Met Val Glu Asn Ser Lys Phe Phe Thr Lys His Gly Ile
               420                 425                 430
   CGA AAT GGG ATC AAC CCT GCC ATG TAC CAG AGC CCA GTA AGG CTC CGA      1344
   Arg Asn Gly Ile Asn Pro Ala Met Tyr Gln Ser Pro Val Arg Leu Arg
           435                 440                 445
   AGT TCA ACC ATT CAG TCT TCA TCC ATC ATT CTC CTC AAT ACA TTT CTC      1392
   Ser Ser Thr Ile Gln Ser Ser Ser Ile Ile Leu Leu Asn Thr Phe Leu
       450                 455                 460
   ACT GAA AAC GAT GGT GAC AAA GTA GAA GAC CAA GTC AGC TAC ATA          1437
   Thr Glu Asn Asp Gly Asp Lys Val Glu Asp Gln Val Ser Tyr Ile
   465                 470                 475
(2) INFORMATION FOR SEQ ID NO:2:
   (i) SEQUENCE CHARACTERISTICS:
      (A) LENGTH: 479 amino acids
      (B) TYPE: amino acid
      (D) TOPOLOGY: linear
   (ii) MOLECULE TYPE: protein
   (xi) SEQUENCE DESCRIPTION: SEQ ID NO:2:
   Met Ala Ser Ser Tyr Lys Met Ser Glu Gln Ser Thr Ile Ser Glu His
    1               5                  10                  15
   Ile Leu Gln Lys Thr Cys Asp His Leu Ile Leu Thr Asp Arg Ser Gly
               20                  25                  30
   Leu Lys Ala Glu Ser Ala Ala Glu Glu Met Lys Gln Thr Ala Glu Asn
           35                  40                  45
   Gln Gly Asn Thr Val His Trp Ala Ala Leu Leu Ile Phe Ala Val Ile
       50                  55                  60
   Ile Pro Thr Ile Gly Gly Asn Ile Leu Val Ile Leu Ala Val Ser Leu
   65                  70                  75                  80
   Glu Lys Arg Leu Gln Tyr Ala Thr Asn Tyr Phe Leu Met Ser Leu Ala
                   85                  90                  95
   Val Ala Asp Leu Leu Val Gly Leu Phe Val Met Pro Ile Ala Leu Leu
               100                 105                 110
   Thr Ile Met Phe Glu Ala Thr Trp Pro Leu Pro Leu Ala Leu Cys Pro
               115                 120                 125
   Ala Trp Leu Phe Leu Asp Val Leu Phe Ser Thr Ala Ser Ile Met His
       130                 135                 140
   Leu Cys Ala Ile Ser Leu Asp Arg Tyr Ile Ala Ile Lys Lys Pro Ile
   145                 150                 155                 160
   Gln Ala Asn Gln Cys Asn Ser Arg Thr Thr Ala Phe Val Lys Ile Thr
                   165                 170                 175
   Val Val Trp Leu Ile Ser Ile Gly Ile Ala Ile Pro Val Pro Ile Lys
               180                 185                 190
   Gly Ile Glu Ala Asp Val Val Asn Ala His Asn Ile Thr Cys Glu Leu
           195                 200                 205
   Thr Lys Asp Arg Phe Gly Ser Phe Met Leu Phe Gly Ser Leu Ala Ala
       210                 215                 220
   Phe Phe Ala Pro Leu Thr Ile Met Ile Val Thr Tyr Phe Leu Thr Ile
   225                 230                 235                 240
   His Ala Leu Arg Lys Lys Ala Tyr Leu Val Arg Asn Arg Pro Pro Gln
                   245                 250                 255
   Arg Leu Thr Arg Trp Thr Val Ser Thr Val Leu Gln Arg Glu Asp Ser
               260                 265                 270
   Ser Phe Ser Ser Pro Glu Lys Met Ala Met Leu Asp Gly Ser His Lys
           275                 280                 285
   Asp Lys Ile Leu Pro Asn Ser Ile Asp Glu Thr Leu Met Arg Arg Met
       290                 295                 300
   Ser Ser Ala Gly Lys Lys Pro Ala Gln Thr Ile Ser Asn Glu Gln Arg
   305                 310                 315                 320
   Ala Ser Lys Val Leu Gly Ile Val Phe Leu Phe Phe Leu Leu Met Trp
                   325                 330                 335
   Cys Pro Phe Phe Ile Thr Asn Val Thr Leu Ala Leu Cys Asp Ser Cys
               340                 345                 350
   Asn Gln Thr Thr Leu Lys Thr Leu Leu Gln Ile Phe Val Trp Val Gly
           355                 360                 365
   Tyr Val Ser Ser Gly Val Asn Pro Leu Ile Tyr Thr Leu Phe Asn Lys
       370                 375                 380
   Thr Phe Arg Glu Ala Phe Gly Arg Tyr Ile Thr Cys Asn Tyr Gln Ala
   385                 390                 395                 400
   Thr Lys Ser Val Lys Val Leu Arg Lys Cys Ser Ser Thr Leu Tyr Phe
                   405                 410                 415
   Gly Asn Ser Met Val Glu Asn Ser Lys Phe Phe Thr Lys His Gly Ile
               420                 425                 430
   Arg Asn Gly Ile Asn Pro Ala Met Tyr Gln Ser Pro Val Arg Leu Arg
           435                 440                 445
   Ser Ser Thr Ile Gln Ser Ser Ser Ile Ile Leu Leu Asn Thr Phe Leu
       450                 455                 460
   Thr Glu Asn Asp Gly Asp Lys Val Glu Asp Gln Val Ser Tyr Ile
   465                 470                 475
(2) INFORMATION FOR SEQ ID NO:3:
   (i) SEQUENCE CHARACTERISTICS:
      (A) LENGTH: 141 base pairs
      (B) TYPE: nucleic acid
      (C) STRANDEDNESS: double
      (D) TOPOLOGY: linear
   (ii) MOLECULE TYPE: cDNA
   (iii) HYPOTHETICAL: NO
   (iv) ANTI-SENSE: NO
   (ix) FEATURE:
      (A) NAME/KEY: CDS
      (B) LOCATION: 1 - 141
   (xi) SEQUENCE DESCRIPTION: SEQ ID NO:3:
   ACC ATT          6
   Thr Ile
    1
   CAC GCT TTG CGG AAG AAA GCT TAC TTG GTC AGA AAC AGG CCA CCT CAA          54
   His Ala Leu Arg Lys Lys Ala Tyr Leu Val Arg Asn Arg Pro Pro Gln
           5                  10                  15
   CGC CTA ACA CGG TGG ACT GTG TCC ACA GTT CTC CAA AGG GAA GAC TCA         102
   Arg Leu Thr Arg Trp Thr Val Ser Thr Val Leu Gln Arg Glu Asp Ser
       20                  25                  30
   TCC TTT TCA TCA CCA GAA AAG ATG GCG ATG CTG GAT GGC                     141
   Ser Phe Ser Ser Pro Glu Lys Met Ala Met Leu Asp Gly
    35                 40                  45
(2) INFORMATION FOR SEQ ID NO:4:
   (i) SEQUENCE CHARACTERISTICS:
      (A) LENGTH: 165 base pairs
      (B) TYPE: nucleic acid
      (C) STRANDEDNESS: double
      (D) TOPOLOGY: linear
   (ii) MOLECULE TYPE: cDNA
   (iii) HYPOTHETICAL: NO
   (iv) ANTI-SENSE: NO
   (ix) FEATURE:
      (A) NAME/KEY: CDS
      (B) LOCATION: 1..165
   (xi) SEQUENCE DESCRIPTION: SEQ ID NO:4:
   ATG GCT TCA TCT TAT AAA ATG TCT GAA CAA AGC ACA ATT TCT GAG CAC          48
   Met Ala Ser Ser Tyr Lys Met Ser Glu Gln Ser Thr Ile Ser Glu His
     1             5                  10                  15
   ATT TTG CAG AAA ACA TGT GAT CAC CTG ATC TTG ACT GAC CGT TCT GGA        96
   Ile Leu Gln Lys Thr Cys Asp His Leu Ile Leu Thr Asp Arg Ser Gly
               20                  25                  30
   TTA AAG GCA GAA TCA GCA GCA GAG GAA ATG AAG CAG ACT GCC GAG AAC       144
   Leu Lys Ala Glu Ser Ala Ala Glu Glu Met Lys Gln Thr Ala Glu Asn
           35                  40                  45
   CAG GGG AAT ACA GTG CAC TGG                                           165
   Gln Gly Asn Thr Val His Trp
       50                  55
(2) INFORMATION FOR SEQ ID NO:5:
   (i) SEQUENCE CHARACTERISTICS:
      (A) LENGTH: 405 base pairs
      (B) TYPE: nucleic acid
      (C) STRANDEDNESS: double
      (D) TOPOLOGY: linear
   (ii) MOLECULE TYPE: cDNA
   (iii) HYPOTHETICAL: NO
   (iv) ANTI-SENSE: NO
   (ix) FEATURE:
      (A) NAME/KEY: CDS
      (B) LOCATION: 1 - 405
   (xi) SEQUENCE DESCRIPTION: SEQ ID NO:5:
   ACT TTA GCT CTG TGT GAT TCC TGC        24
   Thr Leu Ala Leu Cys Asp Ser Cys
   1                   5
   AAC CAG ACT ACT CTC AAA ACA CTC CTG CAG ATA TTT GTG TGG GTA GGC        72
   Asn Gln Thr Thr Leu Lys Thr Leu Leu Gln Ile Phe Val Trp Val Gly
       10                  15                  20
   TAC GTT TCC TCG GGA GTG AAT CCT TTG ATC TAT ACC CTC TTC AAT AAG       120
   Tyr Val Ser Ser Gly Val Asn Pro Leu Ile Tyr Thr Leu Phe Asn Lys
   25                  30                  35                  40
   ACA TTT CGG GAA GCA TTT GGC AGG TAC ATC ACC TGC AAT TAC CAG GCC       168
   Thr Phe Arg Glu Ala Phe Gly Arg Tyr Ile Thr Cys Asn Tyr Gln Ala
                   45                  50                  55
   ACA AAG TCA GTA AAA GTG CTT AGA AAG TGT TCT AGT ACA CTC TAT TTT       216
   Thr Lys Ser Val Lys Val Leu Arg Lys Cys Ser Ser Thr Leu Tyr Phe
               60                  65                  70
   GGG AAT TCA ATG GTA GAA AAC TCT AAA TTT TTC ACA AAA CAT GGA ATT       264
   Gly Asn Ser Met Val Glu Asn Ser Lys Phe Phe Thr Lys His Gly Ile
           75                  80                  85
   CGA AAT GGG ATC AAC CCT GCC ATG TAC CAG AGC CCA GTA AGG CTC CGA       312
   Arg Asn Gly Ile Asn Pro Ala Met Tyr Gln Ser Pro Val Arg Leu Arg
       90                  95                  100
   AGT TCA ACC ATT CAG TCT TCA TCC ATC ATT CTC CTC AAT ACA TTT CTC     360
   Ser Ser Thr Ile Gln Ser Ser Ser Ile Ile Leu Leu Asn Thr Phe Leu
   105                 110                 115                 120
   ACT GAA AAC GAT GGT GAC AAA GTA GAA GAC CAA GTC AGC TAC ATA      405
   Thr Glu Asn Asp Gly Asp Lys Val Glu Asp Gln Val Ser Tyr Ile
                   125                 130                 135

## Description

This invention relates to a novel serotonin receptor protein, and to novel nucleic acid compounds which encode the novel protein.

Since the discovery of serotonin (5-hydrox-ytryptamine, 5-HT) over four decades ago, the cumulative results of many diverse studies have indicated that 5-HT plays a significant role in the functioning of the mammalian body, both in the central nervous system (CNS) and in peripheral systems as well. Morphological studies of the CNS have shown that serotonergic neurons, which originate in the brain stem, form a very diffuse system that projects to most areas of the brain and spinal cord. O'Brien, R.A.: Serotonin in MentalAbnormalities 1, 41 (1978); Steinbusch, H.W.M., Vol. 3, Part II, Handbook of Chemical Neuroanatomy68 (1984). Anden, N.E. et al., 67 Acta Physiol. Scand. 313 (1966). These studies have been complemented by biochemical evidence that indicates large concentrations of 5-HT exists in the brain and spinal cord. Steinbusch, H.W.M., Vol. 3, Part II Handbook of Chemical Neuroanatomy, 68 (1984). With such a diffuse system, it is not surprising that 5-HT has been implicated as being involved in the expression of a number of behaviors, physiological responses, and diseases which originate in the CNS. These include such diverse areas as sleeping, eating, perceiving pain, controling body temperature, controling blood pressure, depression, schizophrenia, and other bodily states. Essman, W.B. Vol. I-V Serotonin in Health and Disease,; Fuller, R.W., Biology of Serotonergic Transmission, 221 (1982); Boullin, D.J., Serotonin in MentalAbnormalities 1, 316 (1978); Barchas, J. et al. Serotonin and Behavior 1 (1973).

Serotonin plays an important role in peripheral systems as well. For example, approximately 90% of the body's 5-HT is found in the gastrointestinal system, and 5-HT has been found to mediate a variety of contractile, secretory, and electrophysiologic effects in this system. Another example of a peripheral network that is very sensitive to serotonin is the cardiovascular system, which also contains its own source of 5-HT, i.e., the platelet. Given the broad distribution of 5-HT within the body, it is understandable that tremendous interest in drugs that affect serotonergic systems exists. In particular, receptor specific agonists and antagonists are of interest for the treatment of a wide range of disorders, including anxiety, depression, hypertension, migraine, compulsive disorders, and cancer-chemotherapy-induced vomiting. Gershon M.D. et al., The Peripheral Actions of 5-hydroxytryptamine, 246 (1989); Saxena, P.R. et al. 15 J. Cardiovasc. Pharmacol. (Suppl. 7 1990).

Serotonin produces its effects on cellular physiology by binding to specialized receptors on the cell surface. It is now recognized that multiple types of 5-HT receptors exist. The existence of multiple, structurally distinct serotonin receptors has provided the possibility that subtypeselective pharmacologic agents can be produced. The development of such compounds could result in new and increasingly selective therapeutic agents with fewer side effects, since individual receptor subtypes may function to affect specific actions of the different parts of the central peripheral serotonergic systems. An example of such specificity can be demonstrated by using the vascular system as an example: In certain blood vessels, stimulation of 5-HT_{I}-like receptors on the endothelial cells produces vasodilation while stimulation of 5-HT₂ receptors on the smooth muscle cells produces vasoconstriction. Currently, the major classes of serotonin receptors (5-HTᵢ, 5-HT₂, 5-HT₃, and 5-HT₄) contain some eight to ten separate receptors that have been formally classified based on their pharmacologic and/or structural differences. For an excellent review of the pharmacological effects and clinical implications of the various 5-HT receptor types, see Glennon, et al. 14 Neuroscience and Behavioral Reviews 35 (1990). The discovery of novel serotonin receptors provides a necessary research tool for the development of selective pharmaceutical agents.

According to the present invention there is provided a novel 5-HT receptor. The present invention provides an amino acid compound which comprises the isolated amino acid sequence SEQ ID NO:2. In particular, the amino acid compound which is SEQ ID NO:2 is preferred.

The invention also provides a nucleic acid compound that comprises an isolated nucleic acid sequence which encodes for the amino acid compounds provided. Particularly, nucleic acid compounds which are DNA are preferred. Most preferred is the DNA compound SEQ ID NO:1. However, also preferred are those nucleic acid compounds which are sense mRNA.

Also provided by the present invention are recombinant nucleic acid vectors comprising the nucleic acids which encode SEQ ID NO:2. The preferred nucleic acid vectors are those which are DNA. Most preferred are recombinant DNA vectors which comprise the isolated DNA sequence which is SEQ ID NO:1. Moreover, recombinant DNAvectors of the present invention preferably comprise a promoter positioned to drive expression of said isolated DNA sequence. Those vectors wherein said promoter functions in AV12 cells or E.coli are preferred. The recombinant DNA expression vector most preferred is plasmid pHD5HT_{zf}. Restriction fragments of the preferred vectors are also provided. Particularly, the 1598 base pair Bgll/Hgal and the 1169 base pairApa-LI/Banl restriction fragment of a vector which comprises SEQ ID NO:1 are provided.

The present invention also provides probes and primers useful for molecular biology techniques. A compound which encodesforSEQ ID NO:2 or a part thereof and which is at least 20 base pairs in length is provided. Preferably, the 20 base pair or more compound is DNA. Most preferred for this use are the DNA compounds which are SEQ ID NO:3, SEQ ID NO:4 and SEQ ID NO:5.

Further, this invention provides cells in which the nucleic acid compounds of the invention may be harbored. An oocyte expressing nucleic acid compounds of the invention is provided. One preferred nucleic acid compound which may be harbored in the oocyte is DNA. Most preferred, however, is an oocyte which expresses sense mRNA. Other host cells include those that are transfected with a nucleic acid compound which encodes SEQ ID NO:2. Preferred cells include a host cell transfected with a recombinant DNA vector. Preferably, the DNAvector comprises SEQ ID NO:1. The preferred transfected host cells which encode SEQ ID NO:2areAV12 and E. coli. The most preferred transfected host cells are AV12/pHD5HT_{2f}, E. coli/pSSHT_{2f}oran E. coli cell transfected with a DNA expression vector comprising SEQ ID NO:1.

Additionally, the invention provides a method for identifying, in a test sample, DNA homologous to a probe of the present invention, wherein the test nucleic acid is contacted with the probe under hybridizing conditions and identified as being homologous to the probe. The preferred probes for use in this method are SEQ ID NO:3, SEQ ID NO:4 or SEQ ID NO:5.

Assays utilizing the compounds provided by the present invention are also provided. Generally, an assay provided includes a method for determining whether a substance is a functional ligand for the compound of SEQ ID NO:2, said method comprising contacting a functional compound of SEQ ID NO:2 with said substance, monitoring serotonergic activity by physically detectable means, and identifying those substances which effect a chosen response.

Preferably, the physically detectable means is competition with radiolabeled serotonin, hydrolysis of phosphatidylinositol or binding of ligand in an oocyte expression system. Most preferred is an assay wherein the radiolabeled serotonin is tritiated at the 3 position or wherein the oocyte expression system utilizes sense mRNA.

The invention also provides a method for constructing a recombinant host cell capable of expressing a nucleic acid compound which encodes a compound which comprises SEQ ID NO:2, said method comprising transfecting a host cell with a recombinant DNA vector that comprises said nucleic acid compound. The preferred method utilizes AV12 or E coli cells as the host cells. The preferred method may include a DNAvector which comprises SEQ ID NO:1. The most preferred method utilizes the DNAvector pHD5HT_{zf}.

Additionally, a method for expressing a nucleic acid sequence which encodes SEQ ID NO:2 in a recombinant host cell is provided. The method comprises culturing a transformed host cell provided by the present invention under conditions suitable for gene expression. The preferred method utilizes AV12 or E. coli cells as the host cell. The more preferred method utilizes a recombinant DNA vector comprising SEQ ID NO:1. The most preferred method utilizes the recombinant DNA vector pHD5HT_{zf}.

The following section provides a more detailed description of the present invention. For purposes of clarity and as an aid in understanding the invention, as disclosed and claimed herein, the following items are defined below.

5-HT_{zf} receptor - the amino acid sequence SEQ ID NO:2.

Amino terminal encoding region - The DNA compound which is SEQ ID NO:4.

Carboxy terminal encoding region - The DNA compound which is SEQ ID NO:5.

Functional compound of SEQ ID NO:2 - A compound comprising SEQ ID NO:2 which is capable of binding serotonin.

G loop encoding region - The DNA compound which is SEQ ID NO:3.

Homologous - fragments or complete nucleic acid compounds which will hybridize to other fragments or complete nucleic acid compounds.

Hybridization - physically detectable adherence of fragments or complete nucleic acids to other fragments or complete nucleic acids.

Isolated amino acid sequence - any amino acid sequence, however constructed or synthesized, which is locationally distinct from the naturally occurring sequence.

Isolated DNA sequence - Any DNA sequence, however constructed or synthesized, which is locationally distinct from its natural location in genomic DNA.

Isolated nucleic acid sequence - Any RNA or DNA sequence, however constructed or synthesized, which is locationally distinct from its natural location.

Primer - a nucleic acid fragment which functions as initial template for enzymatic or synthetic elongation.

Promoter - a DNA sequence which directs transcription of DNA to RNA.

Probe - a nucleic acid compound or a fragment thereof which hybridizes with a nucleic acid compound which encodes either the entire sequence SEQ ID NO:2 or a part thereof.

Stringency - hybridization condition which may be varied in order to vary the degree of nucleic acid affinity for other nucleic acid.

Transfection - any transfer of nucleic acid into a host cell, with or without integration of said nucleic acid into genome of said host cell.

Figure I is a restriction map of pSSHT_{2f}, the recombinant DNAvector discussed herein. The restriction site information is not exhaustive; therefore, there may be more restriction sites of a given type on the vector than actually shown on the map.

The present invention provides an amino acid compound which comprises the isolated amino acid sequence SEQ ID NO:2. The preferred amino acid compound is SEQ ID NO:2, which is the following sequence of amino acids:

Skilled artisans will recognize that this protein can be synthesized by a number of different methods. All of the amino acid compounds of the invention can be made by chemical methods well known in the art, including solid phase peptide synthesis or recombinant methods. Both methods are described in U.S. Patent 4,617,149, herein incorporated by reference. Recombinant methods are preferred if a high yield is desired. A general method for the construction of any desired DNA sequence is provided in Brown et al., 68 Methods in Enzymology 109 (1979). Skilled artisans also recognize that some alterations of SEQ ID NO:2 will fail to change the function of the amino acid compound. For instance, some hydrophobic amino acids may be exchanged for other hydrophobic amino acids. Those altered amino acid compounds which confer substantially the same function in substantially the same manner as the exemplified amino acid compound are also included in the present invention. Moreover, alterations of SEQ ID NO:2 may be induced by alterations of a nucleic acid compound which encodes SEQ ID NO:2. Those nucleic acid compounds which confer substantially the same function in substantially the same manner as the exemplified nucleic acid compounds are also included in the present invention.

Other routes of production are well known to skilled artisans. Expression in eucaryotic cells can be achieved via SEQ ID NO:1. For example, the amino acid compounds can be produced in eucaryotic cells using SV40-derived expression vectors comprising DNA which encodes for SEQ ID NO:2. As well known in the art, some viruses are also appropriate vectors. For example, the adenovirus, the adeno associated virus, the vaccinia virus, the herpes virus, the baculovirus, and the rous sarcoma virus are useful. Such a method is described in U.S. Patent 4,775,624, herein incorporated by reference. Several alternate methods of expression are described in J. Sambrook, E.F. Fritsch & T. Maniatis, Molecular Cloning: A Laboratory Manual 16.3-17.44 (1989).

Other embodiments of the present invention are nucleic acid compounds which comprise isolated nucleic acid sequences which encode SEQ ID NO:2. As skilled artisans will recognize, the amino acid compounds of the invention can be encoded by a multitude of different nucleic acid sequences because most of the amino acids are encoded by more than one nucleic acid triplet. Because these alternate nucleic acid sequences would encode the same amino acid sequences, the present invention further comprises these alternate nucleic acid sequences. Preferably, the nucleic acid compound is DNA or sense mRNA. A most preferred embodiment of a DNA compound encoding the 5-HT_{zf} compound has this sequence:

E. coli/pSSHT_{2f}, which contains a cloning vector comprising SEQ ID NO:1, was deposited and made part of the stock culture collection of the Northern Regional Research Laboratories (NRRL), Agricultural Research Service, U.S. Department of Agriculture, Peoria, Illinois, 61604 on March 24, 1992, under the accession number NRRL B18952. SEQ ID NO:1 can be isolated from the plasmid, for example, as a 1169 base (1.2 kb) Apa-LI/Banl restriction fragment. Otherfragments are useful in obtaining SEQ ID NO:1. Arestriction site and function map of plasmid pSSHT_{2f} is presented in Figure I of the accompanying drawings.

Additionally, the DNA sequences can be synthesized using automated DNAsynthesizers, such astheABS (Applied Biosystems, 850 Lincoln Centre Drive, Foster City, CA 94404) 380B DNA synthesizer. The DNA sequences can also be generated by the polymerase chain reaction as described in U.S. Patent No. 4,889,818, herein incorporated by reference.

Because skilled artisans will recognize that many vectors are available for expression and cloning, those expression and cloning vectors which comprise nucleic acids which encode SEQ ID NO:2 are included in the present invention. The preferred nucleic acid vectors are those which are DNA. The most preferred recombinant DNA vector comprises the isolated DNA sequence SEQ ID NO:1. Plasmid pSSHT_{2f} is a preferred DNA vector of the present invention.

Other preferred DNA vectors include those which comprise a promoter positioned to drive expression of SEQ ID NO:1. Preferred expression vectors include those which function in AV12 or E. coli cells. Most preferred for expression in AV12 cells is the expression vector pHD5HT_{2f}.

Restriction fragments of these vectors are also provided. The preferred fragments are the 1598 base pair Bgll/Hgal restriction fragment and the 1169 base pair ApaLl/Banl restriction fragment of a vector which comprises SEQ ID NO:1.

Plasmid pS5HT2f may be isolated from the deposited E coli/pSSHT_{2f}, using an ordinary cesium chloride DNA isolation procedure. Plasmid pS5HT_{2f} is readily modified to construct expression vectors that produce 5-HT_{2f} receptors in a variety of organisms, including, for example, E coli, Sf9 (as host for baculovirus), Spodop- tera and Saccharomyceyes. The current literature contains techniques for constructing AV12 expression vectors and for transforming AV12 host cells. For example, U.S. Patent No. 4,992,373, herein incorporated by reference, explains these techniques.

The construction protocols utilized for AV12 can be followed to construct analogous vectors for other organisms, merely by substituting, if necessary, the appropriate regulatory elements using techniques well known to skilled artisans. Promoters which may be used, for example, are the thymidine kinase promoter, the metallothionin promoter or various viral and immunoglobulin promoters.

The DNA compounds of the present invention also include primers or probes. A nucleic acid compound of at least 20 base pairs which encodes SEQ ID NO:2 is included in the present invention. DNA is the preferred nucleic acid used as a probe or primer. Most preferred DNAcompounds useful as probes or primers are: SEQ ID NO:3, SEQ ID NO:4 and SEQ ID NO:5. A skilled artisan would recognize the techniques associated with probes and primers as well known in the art. Any sequence of at least 20 base pairs in length of the nucleic acids of the present invention may be used to screen any other nucleic acid. For example, all or part of SEQ ID NO:4 or SEQ ID NO:5 may be used to hybridize to the terminal ends of the coding sequence. Then, through PCR amplification, the full length sequence may be generated. The full length sequence can be subsequently subcloned into any vector of choice.

Alternatively, SEQ ID NO:3, SEQ ID NO:4, or SEQ ID NO:5 may be radioactively labeled at the 5' end in order to screen cDNA libraries by conventional means. Furthermore, any piece of 5-HT_{2f} DNAwhich has been bound to a filter may be flooded with total mRNA transcripts, in order to then reverse-transcribe the mRNA transcripts which bind.

Primers and probes may be obtained by means well known in the art. For example, once pS5HT2f is isolated, restriction enzymes and subsequent gel separation may be used to isolate the fragment of choice.

Host cells which harbor the nucleic acids provided by the present invention are also provided. A preferred host cell is an oocyte which has been injected with RNA or DNA compounds of the present invention. Most preferred oocytes of the present invention are those which harbor sense mRNA. Other preferred host cells include AV12 and E. coli cells which have been transfected and/or transformed with a vector which comprises SEQ ID NO:1. Most preferred AV12 and E. coli host cells are AV12/pHD5HT_{2f}, E coli/pSSHT_{2f}, and an E. colilE. coli expression vector which comprises SEQ ID NO:1.

The oocyte expression system can be constructed according to the procedure described in Lübbert, et al. 84 Proc. Nat. Acad. Sci. 4332 (1987). Other host cell transfection and transformation is well known in the art.

Also provided by the present invention is a method for expressing a gene which encodes SEQ ID NO:2 in a recombinant host cell, said method comprising culturing said transformed host cell under conditions suitable for gene expression. The preferred method utilizes AV12 or E. coli cells. The most preferred method utilizes AV12 cells as host cells for pHD5HT_{2f} or E. coli cells as host cells for a recombinant DNA vector comprising SEQ ID N0:1. Expression in host cells may be accomplished according to the procedures outlined in Sambrook, et al., Molecular Cloning: A Laboratory Manual 16-17 (1989).

The present invention also provides a method for constructing a recombinant host cell capable of expressing SEQ ID NO:2, said method comprising transforming a host cell with a recombinant DNA vector that comprises an isolated DNA sequence which encodes SEQ ID NO:2. The preferred host cell is AV12, which may be obtained from the ATCC under the accession numberATCC CRL 9595. The preferred vector for expression is one which comprises SEQ ID NO:1. Especially preferred for this purpose is pHD5HT_{zf}. Another preferred host cell for this method is E coli. An especially preferred expression vector in E. coli is one which comprises SEQ ID NO:1. Transformed host cells may be cultured under conditions well known to skilled artisans such that SEQ ID NO:2 is expressed, thus producing 5-HT_{2f} activity in the recombinant host cell.

Additionally, the invention provides a method for identifying, in a test sample, DNA homologous to a probe of the present invention, wherein the test nucleic acid is contacted with the probe under hybridizing conditions and identified as being homologous to the probe The preferred probes for use in this method are SEQ ID NO:3, SEQ ID NO:4 or SEQ ID NO:5. Hybridization techniques are well known in the art. Sambrook, et al., Molecular Cloning: A Laboratory Manual 11 (1989) describe such procedures.

Assays utilizing the compounds provided by the present invention are also provided. Generally, an assay provided includes a method for determining whether a substance is a functional ligand for the compound of SEQ ID NO:2, said method comprising contacting a functional compound of SEQ ID NO:2 with said substance, monitoring serotonergic activity by physically detectable means, and identifying those substances which effect a chosen response. Preferably, the physically detectable means is competition with radiolabeled serotonin, hydrolysis of phosphatidylinositol or binding of ligand in an oocyte expression system. Most preferred is an assay wherein the radiolabeled serotonin is tritiated at the 3 position or wherein the oocyte expression system utilizes sense mRNA.

The oocyte expression system can be constructed according to the procedure described in Lübbert, et al. 84 Proc. Nat. Acad. Sci. 4332 (1987). The radiolabeled 5-HT competition assay may be accomplished according to Nelson, et al., 41 Life Sciences 1567 (1567). The assay which measures serotonergic activity via phosphatidylinositol hydrolysis may be accomplished according to Berridge M., 212 Biochem. J. 849 (1983).

Skilled artisans will recognize that desirable K, values are dependent on the selectivity of the compound tested. For example, a compound with a K, which is less than 10 nM is generally considered an excellent candidate for drug therapy. However, a compound which has a lower affinity, but is selective for the particular receptor, may be an even better candidate. The present invention, however, provides radiolabeled competition assays, whether results therefrom indicate high affinity or low affinity to 5-HT_{2f} receptor, because skilled artisans will recognize that any information regarding binding or selectivity of a particular compound is beneficial in the pharmaceutical development of drugs.

The following are examples of the present invention:

### Example 1

### Production of 5-HT_{2f} receptor in AV12/pHDSHT_{2f} cells

A lyophil of E coli pS5HT_{2f} can be obtained from the Northern Regional Research Laboratories, Peoria, IL 61604 under the accession number NRRL B18952 and used directly as the culture in the process described below.

Plasmid pS5HT_{2f} was isolated from E coli/pS5HT_{2f} by procedures well known by those skilled in the art. Once pSSHT_{2f} was isolated from the deposited strain, the plasmid was altered using conventional methods to produce a Sall restriction site in addition to the preexisting Sall restriction site. The plasmid was then digested with SnaB1 restriction enzyme to produce a blunt end of DNA. The resulting fragment was linear. Ligase was used to ligate a Sall linker sequence to the blunt end created by SnaB1. The plasmid was then recircularized with ligase to produce two Sail endonuclease sites at both flanking regions of SEQ ID NO:1. Afterthe plasmid was recircularized, the plasmid was digested with Sail restriction endonuclease and purified by gel purification and subsequent extraction. The fragment comprising SEQ ID N0:1 was then inserted into a modified pHD vector. The modified pHD vector is substantially the vector described in issued Patent No. 4,992,373, except that the plasmid is cleaved at the Bcl site, shortened by Klenow to form blunt ends, and ligated with a Sall linker to form a Sall restriction site. The resulting pHD vector contained a Sail restriction site, an adenovirus promoter, and an ampicillin resistance marker to provide selection in E. coli. The pHD vector was dephosphorylated in order to prevent recircularization. Subsequently, the fragment comprising SEQ ID NO:1 was ligated into the pHD vector. Competent E coli cells were then transformed with the pHD vector containing SEQ ID NO:1 and selected forthose cells which contained the ampicillin resistance gene by growing on ampicillin-containing medium. At this point, restriction digests were done to determine orientation of the gene. After transformation of the pHD vector into E coli, a subsequent plasmid preparation was made in order to isolate the pHDSHT_{2f}vector. In order to transfect AV12 cells with the pHD5HT_{2f} vector, the procedure according to issued Patent No. 4,992,373 was used. Selection on hygromycin was included in this step. After selection on hygromycin, a pure culture was obtained by picking the colonies which grew in the presence of hygromycin. These colonies were then assayed to determine serotonin binding ability.

### Example 2

### Binding Assay for the 5-HT _{2f} Receptor

The 5-HT_{2f} receptor was defined by radioligand binding according to the following procedure:

### A. Tissue Preparation

AV12/5-HT_{2f} cells which had been grown in suspension were centrifuged at 1000 g to remove the growth medium, and the resulting pellet was washed by resuspending (using vortexing) in ice-cold Tris(Sigma) -HCI buffer (50 mM, pH 7.4). The cells were then centrifuged at 39,800 g for 10 minutes. This washing procedure was repeated for a total of three times with a 10 minute incubation at 37°C between the first and second washes. The final pellet was resuspended in ice-cold Tris-HCI buffer (67 mM, pH 7.4) to achieve a final concentration of approximately 2.5-9 million cells per 200 µl, depending on the degree of receptor expression in the cells. The final resuspension was effected by using a Tissumizer (Tekmar) at setting 65, for 15 seconds, to thoroughly disrupt the cell membranes.

### B. Saturation Assay to Determine Affinity for [³H]5-HT

Tubes for the saturation binding assay had a final volume of 800 µl and contained the following: Tris-HCI (500 mM), pargyline (10 µM), ascorbic acid (5.7 mM), CaC1₂ (3 mM), various concentrations of [³H]5-HT (concentrations ranged from approximately 0.5 to 35 nM), and 200 µl of cell membrane resuspension for a final pH of 7.4. The assay tubes were incubated for 15 minutes at 37°C (or in some cases 2 hours at 0°C), and the contents were then rapidly filtered through GF/B filters (Whatman) (pretreated with 0.5% polyethylenimine), and followed by four 1 ml washes with ice-cold buffer (50 mM Tris-HCI, pH 7.4). The radioactivity trapped by the filters was quantitated by liquid scintillation spectrometry, and specific [³H]5-HT binding was defined as the difference between [³H]5-HT bound in the presence and absence of 10 µM unlabeled 5-HT. The affinity of [³H]5-HT for the 5-HT_{2f} receptor was calculated from the saturation curves by use of nonlinear regression analysis (SYSTAT, SYSTAT, Inc., Evanston, IL). Using this method, K_{d} values calculated for [³H]5-HT at the 5-HT_{2f} receptor ranged from approximately 5-8 nM with a mean of 6.9 nM at 37°C and 0.15-0.3 nM with a mean of 0.19 nM at 0°C. Skilled artisans will recognize the resultant K_{d} as indicative of a serotonergic receptor.

### Example 3

### Competition Binding Assays

### A. [3H]5-HT Assay

To determine the affinities of various other compounds at the 5-HT_{2f} receptor, the assays were carried out as described in Example 2, except that a fixed concentration of [³H]5-HT (2 nM) was used and the tubes contained appropriate dilutions of the drugs of interest, for example methoxytryptamine or ritanserin, in order to generate competition curves.

The EC₅₀ values from the competition curves were determined by nonlinear regression (SYSTAT, SYSTAT, Inc., Evanston, IL) and converted to K, values by use of the Cheng-Prusoff equation discussed in Cheng, Y. et al., 22 Biochem. Pharmacol. 3099 (1973).

### B. Phosphatidylinositol Hydrolysis Assay

Wells of a 12 well plate were seeded with enough AV12/pHDSHT_{2f} cells in 1.0 ml of media containing 4.0 µ Ci of ³H-myoinositol such that confluency was obtained in 48 hours. (for AV12 cells this was 5 x 10⁵ cells). After 48 hours in a tissue culture incubator, the media was aspirated, and the cells were washed two times with serum-free media containing 10 mM myoinositol and 10 mm LiCI. After the final wash, 1.0 ml of the serum free wash media containing 10 mM myoinositol an 10 mM LiCI was added. Dilutions of appropriate compounds were added to the wells and the plate was floated in a 37°C water bath for 1 hour. The reaction was terminated by aspirating the media and adding 0.5 ml of ice-cold acetone:methanol (1:1, v:v). The plate was then left on a rocking platform for 20 minutes at 4°C.

The extract was harvested into an eppendorf tube and the well was rinsed with 0.5 ml water and combined with the extract. Samples were centrifuged in an eppendorf centrifuge for approximately 10 minutes in order to pellet the cellular residue. The supernatants were loaded onto SepPak Waters Accell Plus QMA cartridges which were previously treated by adding 10 ml of 1M ammonium formate, 0.1 M formic acid to the cartridge and then drawing the solution through with vacuum. Next, the cartridge was washed two times with 10 ml water. Five ml water was added, and then the sample was added. The vacuum manifold was then used to draw samples through the cartridges. After the sample went through the cartridges, 10 ml of 5.0 mM sodium tetraborate was added to each cartridge and drawn through.

The manifold was loaded with 20 ml scintillation vials and 4.0 ml 0.1 M ammonium formate, 0.01 M formic acid and 5 mM Na tetraborate was added to the cartridges and the eluates were drawn into the vials using vacuum. Next, 18 ml Ready Solv HP was added to each vial and the radioactive inositol phosphate eluted from the cartridges was quantitated by scintillation spectroscopy. Curves which illustrate the EC₅₀ of 5-HT at various concentrations of the antagonist were generated, and a Shield Plot was used to calculate K_{;}, as accomplished in Berridge M., 212 Biochem. J. 849 (1983).

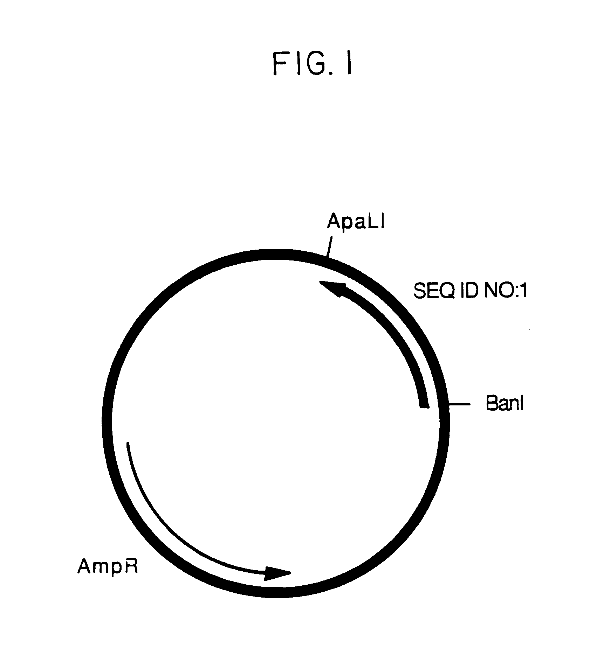

## Claims

1. An amino acid compound which comprises the isolated amino acid sequence SEQ ID NO:2.

2. The amino acid compound of Claim 1 which is SEQ ID NO:2.

3. A nucleic acid compound which comprises an isolated nucleic acid sequence which encodes for the compound of Claim 2.

4. The DNA compound of Claim 4 which is SEQ ID NO:1.

5. A recombinant nucleic acid vector which comprises the nucleic acid compound of Claim 3 or 4.

6. A host cell transfected with a recombinant nucleic acid vector of Claim 5.

7. The transfected host cell of Claim 30 which is E. co/i/pS5MT_{2f}.

8. A method for determining whether a substance is a functional ligand for the compound of SEQ ID NO:2, said method comprising contacting a functional compound of SEQ ID NO:2 with said substance, monitoring serotonergic activity by physically detectable means, and identifying those substances which effect a chosen response.

9. A method for constructing a recombinant host cell capable of expressing the compound of Claim 3, said method comprising transforming a host cell with a recombinant nucleic acid vector that comprises the compound of Claim 3.

10. A method for expressing a nucleic acid sequence which encodes SEQ ID NO:2 in a recombinant host cell, said method comprising culturing said host cell under conditions suitable for gene expression.
